# EUROPEAN PATENT APPLICATION

(11) **EP 3 275 893 A1**
(43) Date of publication of application: **31.01.2018**
(21) Application number: 15886290.4
(22) Date of filing: 23.03.2015
(51) Int. Cl.: C07K 14/435, A61K 38/00, A61L 24/00, C12N 15/09

(54) **ADHESIVE MOLECULE**

(71) Applicant: The Chugoku Electric Power Co., Inc., Hiroshima-shi, Hiroshima 730-8701 (JP); Sessile Research Corporation, Himeji-shi, Hyogo 672-8023 (JP)
(72) Inventor: YOSHIZAKI, Tsukasa, Hiroshima-shi Hiroshima 730-8701 (JP); YAMASHITA, Keiji, Himeji-shi Hyogo 672-8023 (JP); KAMIYA, Kyoko, Himeji-shi Hyogo 672-8023 (JP); SUZUKI, Kohichi, Himeji-shi Hyogo 672-8023 (JP); HAYASHI, Yoshio, Himeji-shi Hyogo 672-8023 (JP)
(74) Representative: J A Kemp
(86) International application number: PCT/JP2015/058770
(87) International publication number: WO 2016/151735

(57) **Abstract**

To provide novel adhesive molecules. An adhesive molecule having an amino acid sequence of SEQ ID NO. 1, or an amino acid sequence including a conservative substitution, a deletion, an insertion and/or a modification in the sequence of SEQ ID NO. 1.

SEQUENCE LISTING

<110>  THE CHUGOKU ELECTRIC POWER CO., INC.
       SESSILE RESEARCH CORPORATION

<120>  adhesive molecule

<130>  K20131200

<160>  2

<170>  PatentIn version 3.5

<210>  1
<211>  126
<212>  PRT
<213>  Artificial Sequence

<220>
<223>  39pr

<400>  1

Cys Gly Ser Lys Ser Tyr Asn Thr Lys Ser His Val Cys Arg Tyr Glu
1               5                   10                  15

Lys Val Trp Pro Lys Ser Tyr Gln Phe Cys Gly Lys Val Gly Tyr Asn
            20                  25                  30

Pro Lys Thr His Val Cys Arg Ser Gly Lys Val Trp Pro Lys Ser Tyr
        35                  40                  45

Lys Phe Cys Gly Asn Val Gly Tyr Asn Pro Lys Thr His Tyr Cys Arg
    50                  55                  60

Ala Gly Arg Ile Tyr Pro Asn His Ile Gln Phe Cys Gly Ser Lys Ser
65                  70                  75                  80

Tyr Asn Thr Lys Ser His Val Cys Arg Tyr Glu Lys Val Trp Pro Lys
                85                  90                  95

Ser Tyr Gln Phe Cys Gly Lys Val Gly Tyr Asn Pro Lys Thr His Val
            100                 105                 110

Cys Arg Ser Gly Lys Val Trp Pro Lys Ser Tyr Lys Phe Cys
        115                 120                 125

<210>  2
<211>  393
<212>  DNA
<213>  Artificial Sequence

<220>
<223>  nucleotide sequence of 39pr with restriction sites

<400> 2
ggatcctgcg gttctaagag ctacaacacc aaaagccatg tgtgtcgcta cgaaaaggtt 60

tggccaaaat cttaccagtt ttgcggtaaa gttgggtata acccgaaaac acacgtttgt 120

cgtagtggaa aagtctggcc taagtcctac aaattctgcg gtaatgtggg ctataatccg 180

aaaactcact attgtcgtgc gggtcgcatt tatccgaatc acatccaatt ttgcggcagt 240

aaatcgtaca acaccaaatc ccatgtatgt cgctatgaga aagtctggcc aaagtcgtat 300

cagttttgcg gcaaagtggg ctataacccc aaaacgcatg tatgtcggtc agggaaagtg 360

tggccgaaaa gctacaaatt ctgctaagaa ttc 393

## Description

### [Technical field]

The present invention relates to adhesive molecules.

### [Background art]

As underwater adhesive molecules derived from organisms, adhesive proteins derived from marine organisms, Balanomorpha and Mytilidae, are disclosed (see, for example, Patent Documents 1 to 3). Adhesive proteins derived from Balanomorpha, however, have not been practically used due to complicated production process that involves, for example, mixing 6 to 8 kinds of proteins to achieve adherence. On the other hand, adhesive proteins derived from Mytilidae are commercially available as bioadhesives containing three kinds of adhesive proteins under the trade name Cell-Tak™, but these proteins are difficult to go into mass production and are thus expensive. Accordingly, discovery of novel adhesive molecules has been awaited.

### [Related art documents]

### [Patent documents]

Patent Document 1: JP-A-8-266282
Patent Document 2: JP-A-2013-226158
Patent Document 3: JP-A-2008-504016

### [Summary of the invention]

### [Problems to be solved by the invention]

An object of the present invention is to provide novel adhesive molecules.

### [Means to solve the problems]

The present inventors found a peptide fragment showing 35% homology with galaxin in peptides that are specifically present in the cement gland of actinula larvae of *Tubularia* sp. Investigations of properties of this newly discovered peptide fragment led to the findings that a peptide having a sequence of SEQ ID NO. 1 including an amino acid sequence of a portion of the peptide fragment had adherence and then the present invention was completed. It should be noted that galaxin is a protein supposed to be involved in the calcification mechanism by which coral skeletons are formed in *Galaxea fascicularis* (see, for example, Fukuda et al., Biochem. Biophys. Res. Commun. 2003 Apr 25; 304(1):11-7) but information related to adherence have not been reported so far on the protein.

The present invention was made to solve at least some of the aforementioned problems and can be implemented as follows.

An aspect of the present invention is an adhesive molecule having an amino acid sequence of SEQ ID NO. 1, or an amino acid sequence of SEQ ID NO. 1 having a conservative amino acid substitution, a deletion, an insertion and/or a modification. The adhesive molecule may contain at least one dihydroxyphenylalanine.

Another aspect of the present invention is an adhesive containing the aforementioned adhesive molecule.

A yet another aspect of the present invention is a polynucleotide having a nucleotide sequence encoding the aforementioned adhesive molecule.

A yet another aspect of the present invention is a vector having the polynucleotide having a nucleotide sequence encoding the aforementioned adhesive molecule.

A still another aspect of the present invention is a method of bonding a first object to a second object, including applying the aforementioned adhesive to a surface of the first object.

A still another aspect of the present invention is a method of coating a surface of an object including applying the adhesive to the surface of the object.

A still another aspect of the present invention is a method of enhancing adherence of an adhesive molecule including reacting tyrosinase with the aforementioned adhesive molecule.

### [Effect of the invention]

The present invention made it possible to provide novel adhesive molecules.

### [Brief description of the drawings]

[Fig. 1] Fig. 1 shows a map of the vector pGEX6P-1 which is used to produce adhesive molecules in microorganisms, according to an embodiment of the present invention.
[Fig. 2] Fig. 2 shows a result of SDS-PAGE of a GST-recombinant peptide fusion product produced in *E. coli,* according to an embodiment of the present invention.
[Fig. 3] Fig. 3 shows adherence of adhesive molecules according to an embodiment of the present invention.

### [Embodiments for carrying out the invention]

Embodiments of the present invention are described in detail along with examples.

Unless otherwise noted in embodiments and examples, all procedures used are according to standard protocols described in, for example, J. Sambrook, E. F. Fritsch & T. Maniatis (Ed.), Molecular cloning, a laboratory manual (3rd edition), Cold Spring Harbor Press, Cold Spring Harbor, New York (2001); F. M. Ausubel, R. Brent, R. E. Kingston, D. D. Moore, J.G. Seidman, J. A. Smith, K. Struhl (Ed.), Current Protocols in Molecular Biology, John Wiley & Sons Ltd., with or without modifications or changes. In addition, commercial reagent kits or measurement instruments are used as described in protocols attached thereto, unless otherwise noted.

The objects, features, advantages, and ideas of the present invention are apparent to those skilled in the art from the description of this specification. Furthermore, those skilled in the art can easily reproduce the present invention from the description herein. The embodiments and specific examples described below represent preferable embodiments of the present invention, which are given for the purpose of illustration or explanation. The present invention is not limited thereto. It is obvious to those skilled in the art that various changes and modifications may be made according to the description of the present specification within the spirit and scope of the present invention disclosed herein.

### (1) Adhesive molecules of the present invention

Adhesive molecules of the present invention have an amino acid sequence of SEQ ID NO. 1 or amino acid sequences including conservative amino acid substitutions, deletions, insertions and/or modifications in the sequence of SEQ ID NO. 1. In the present invention, "conservative amino acid substitutions, deletions, insertions and/or modifications" means substitutions, deletions, insertions and/or modifications that do not impair properties of peptide, that is, do not impair adherence of the adhesive molecules. The adherence as used herein refers to a character of adhering to and settling on a surface of an object without being separated from the surface of the object even after the object is washed, and a molecule is judged to have adherence when a blue band was observed in a test described in Example 2. With such adherence, two objects can be adhered and joined to each other by allowing the two objects to be deposited on adhesive molecules of the present invention simultaneously.

Examples of a conservative amino acid substitution of the present invention include substitutions of an amino acid with another in the same group categorized according to, for example, bulk, polarity, hydrophobicity or hydrophilicity, and acidity or basicity of amino acid residues. Examples of categories are well known to persons skilled in the art and described in various documents (see, for example, Bowie et al., Science, 247:1306-1310 (1990) and Zubay, G., Biochemistry, third edition, Wm. C. Brown Publishers (1993)). For example, amino acids for conservative substitution can be selected based on the groups of amino acids exemplified below. The letters in the parentheses are abbreviations for amino acids in the three letter format. Hydrophilic amino acids (Arg, Asp, Asn, Cys, Glu, Gln, Gly, His, Lys, Ser, and Thr) Hydrophobic amino acids (Ala, Ile, Leu, Met, Phe, Pro, Trp, Tyr, and Val) Amino acids with side chains containing aromatic rings (His, Phe, Tyr, and Trp) Amino acids with aliphatic side chains (Gly, Ala, Val, Leu, Ile, and Pro) Amino acids with side chains containing hydroxyl groups (Ser, Thr, and Tyr) Amino acids with side chains containing sulfur atoms (Cys and Met) Amino acids with side chains containing amides and carboxylic acids (Asp, Asn, Glu, and Gln) Amino acids containing acidic groups (Asp and Glu) Amino acids containing basic groups (Arg, Lys, and His) Polar amino acids (Gln and Asn) Non-polar amino acids (Gly, Ala, Phe, Val, Leu, Ile, Met, Pro, and Trp) Amino acids with small side chains (Gly, Ala, Ser, Thr, and Met) Amino acids with β-branched side chains (Thr, Val, and Ile)

It is also known in the art that peptides having an amino acid sequence including a substitution, a deletion, an insertion and/or a modification of one to several amino acid residues relative to a certain amino acid sequence do not lose their properties (see, for example, Mark, D. F. et al., Proc. Natl. Acad. Sci. USA (1984) 81, 5662-5666, and Dalbadie-McFarland, G. et al., Proc. Natl. Acad. Sci. USA (1982) 79, 6409-6413).

The number of conservative amino acid substitutions, deletions, insertions and/or modifications is not particularly limited as long as the adherence of the adhesive molecules are not impaired, and the number for each may be any number equal to or larger than 1. The number for each is preferably 4 or smaller, more preferably 3 or smaller, yet more preferably 2 or smaller, and most preferably 1 or smaller.

It is preferable that the adhesive molecule of the present invention has the sequence of SEQ ID NO. 1.

It is preferable that the adhesive molecules of the present invention have at least one modified tyrosine residue, dihydroxyphenylalanine (hereinafter, referred to as "DOPA"). In DOPA, the hydrogen atom at position 3 of the benzene ring of the tyrosine residue is replaced with a hydroxyl group. The number of DOPAs contained in an adhesive molecule of the present invention is not particularly limited, but more preferably two, and yet more preferably three. A method of converting a tyrosine residue into DOPA is not particularly limited. For example, molecules having capability of replacement with a hydroxyl group, addition of an oxygen, oxidization can be used. It is preferable that tyrosinase is used.

### (2) Method of producing adhesive molecules of the present invention

A method of producing adhesive molecules of the present invention is not particularly limited and methods of synthesizing them chemically or producing them in cells are examples.

Examples of chemical synthesis include solid phase peptide synthesis such as Boc or Fmoc strategies and liquid phase peptide synthesis, but not limited thereto. It is generally understood that rather shorter peptides can be synthesized more easily and the adhesive molecules of the present invention have a length that allows their easy synthesis.

In a method of producing adhesive molecules in cells, an expression vector, which has a nucleotide sequence encoding an amino acid sequence of an adhesive molecule of the present invention and can express the adhesive molecule, is constructed and introduced into cells to make transformants; and the transformants are allowed to produce the adhesive molecule of the present invention. Methods of constructing an expression vector, introducing it into cells, allowing the cells to produce the protein are known to persons skilled in the art and can be achieved using any one of available methods. The cells are not limited and examples include yeast, insect cells, plant cells, animal cells, and microorganisms. *E. coli* is typically used. The adhesive molecules of the present invention produced in cells can be recovered from the cells or a medium in which the cells have been cultured and then purified using any one of the methods known to persons skilled in the art. The purified adhesive molecules of the present invention can be stored in any methods such as freezing or freeze-drying.

Furthermore, in producing adhesive molecules of the present invention, adherence of adhesive molecules of the present invention may be enhanced under a reaction condition used to convert a tyrosine residue into DOPA, for example, by reacting a tyrosine residue with tyrosinase. In order to achieve a desired adherence, a period of the reaction with tyrosinase can appropriately be determined. The reaction time is not particularly limited but is preferably 10 minutes or longer, more preferably 30 minutes or longer, and yet more preferably 60 minutes or longer.

### (3) Applications of adhesive molecules of the present invention

The adhesive molecules of the present invention can be used as an active ingredient of an adhesive used to bond objects to each other. The objects to be bonded are not particularly limited and examples include glass, polymer compounds such as plastics and resins, metals, woods, or biomaterials. Either objects of the same kind or objects of different kinds can be bonded to each other.

Other component(s) in the adhesive containing the adhesive molecules of the present invention can appropriately be selected to impart, to the adhesive, a desired adhesive strength or a character suitable for an environment where the adhesive is to be used, and vehicles are typical examples. Examples of vehicles include known surfactants, oxidants, and fillers. As alternative examples, known adhesive molecules can be contained.

The concentration of the adhesive molecules of the present invention in the adhesive is not particularly limited and can appropriately be selected from, for example, 1 to 99% (w/v), 5 to 95% (w/v), 10 to 90% (w/v), 20 to 80% (w/v), 30 to 70% (w/v), and 40 to 60% (w/v). The concentration is preferably 0.001% or higher (w/v or v/v), and more preferably 0.01% or higher (w/v or v/v). The form of the adhesive is not particularly limited and may be either in a liquid form or in a solid form. Examples include adhesives in the form of liquid, mist, or gel and adhesive tapes.

The environment where the adhesive molecules of the present invention and/or adhesives made of the adhesive molecules are used is not particularly limited, and an underwater environment (in freshwater or in salt water) or an environment within a living body are examples. For example, they can be used as an underwater adhesive in a civil engineering field, as a dental adhesive (also called an adhesive resin cement) or a hemostatic agent in a living body in a medical field, and as a reagent for regenerative medicine or a component thereof.

The adhesive molecules according to the present invention can be used as a coating agent taking advantage of their character of adhering to a surface of an object. This coating agent imparts adherence to the surface of the object and therefore give character of capturing of moving things (such as microparticles, microorganisms, and large or small organisms) to the object. Since the adhesive molecules of the present invention are not taken off from the surface even by washing, an object can be used as a trap for taking, for example, marine organisms by coating it with the adhesive molecules in water.

### (4) Methods of bonding and coating using adhesive molecules of the present invention

Objects can be bonded using an adhesive containing adhesive molecules of the present invention. For example, objects can be bonded to each other by applying an adhesive of the present invention to a surface of an object using any method such as instillation or coating, placing another object on top of it, and drying it. The amount of the adhesive used for bonding can appropriately be determined depending on a purpose.

As an example of a method of coating a surface of an object using an adhesive containing adhesive molecules of the present invention, the adhesive containing a certain concentration of the adhesive molecules of the present invention can be applied to the surface of the object using any method such as instillation or coating and appropriately be dried.

In methods of bonding and coating, a drying period is not particularly limited but is preferably 5 minutes or longer, more preferably 10 minutes or longer, yet more preferably 30 minutes or longer, and most preferably 60 minutes or longer. Depending on a surface area to be bonded or coated, drying may be performed for a longer period of time.

### [Examples]

### <Example 1> Production of adhesive molecules using E. coli

An non-natural polynucleotide (SEQ ID NO. 2) was generated by determining a nucleotide sequence corresponding to the amino acid sequence of SEQ ID NO. 1 and adding a recognition sequence for the restriction enzyme BamHI and a recognition sequence for the restriction enzyme EcoRI to the 5- and 3-ends, respectively, of the nucleotide sequence.

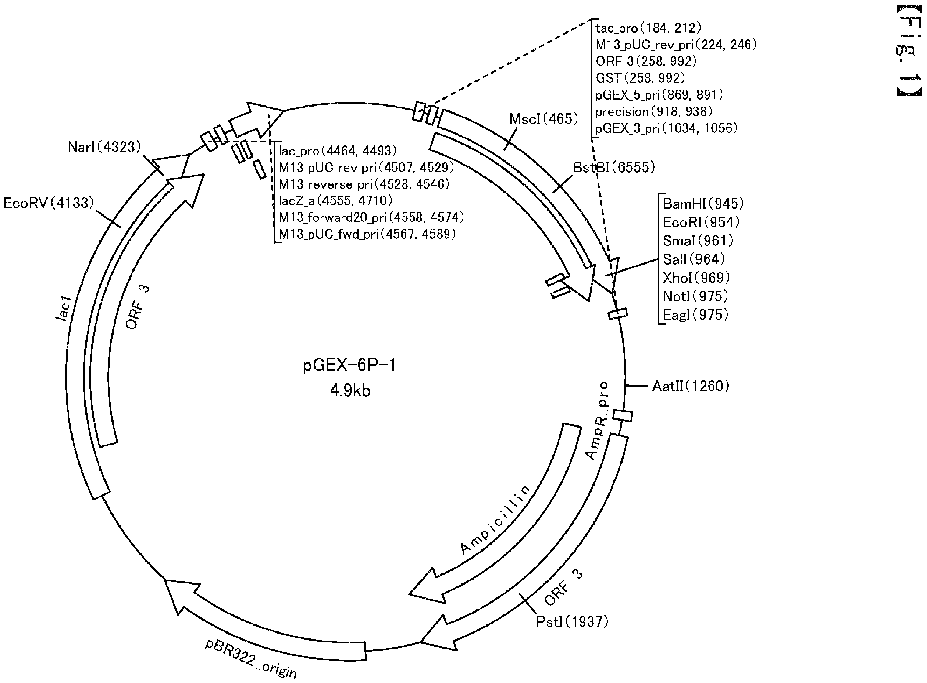

The vector pGEX6P-1 (GE Healthcare) shown in Fig. 1 was digested with the aforementioned restriction enzymes and ligated to the non-natural polynucleotide. A nucleotide sequence generated by insertion of the non-natural polynucleotide into the vector was determined using a sequencing technique. This recombinant vector was introduced into a host *E. coli* (Rosetta-gami B, Merck Millipore) according to the attached protocol to generate a transformant. The vector pGEX6P-1 is formed so that the N-terminus of a peptide to be expressed is fused to Glutathione S-transferase (GST).

The transformant obtained was cultured with shaking at 37°C in an LB medium supplemented with ampicillin and chloramphenicol at a final concentration of 50 µg/ml each, and kanamycin and tetracycline at a final concentration of 15 µg/ml each. When the turbidity (OD600) of the medium reached 0.4 to 0.8, isopropyl-β-thiogalactopyranoside (isopropyl β-D-1-thiogalactopyranoside, IPTG) was added to the LB medium at a final concentration of 0.1 mM in order to induce expression of the GST-fused recombinant peptide. After the addition of IPTG, the temperature was reduced from 37°C to 16°C and the culture with shaking was continued overnight until the turbidity of the LB medium became steady, that is, the growth of *E. coli* became steady. The culture condition of incubation at an IPTG concentration of 0.1 mM at a temperature of 16°C overnight was determined so that the GST-fused recombinant peptide was soluble in a soluble fraction described below.

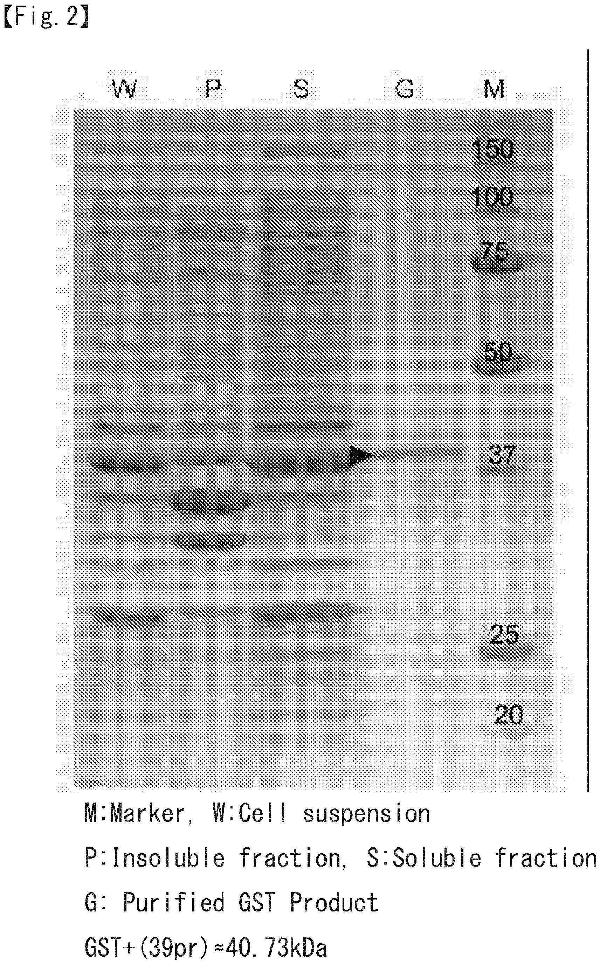

Next, the medium with the transformants was centrifuged (6,000 rpm at 4°C) to remove the supernatant and the precipitates were resuspended in phosphate buffered saline (PBS) to obtain PBS suspension. The PBS suspension was subjected to ultrasonic disruption and then centrifuged at 12,000 rpm and 4°C for 5 minutes to collect the supernatant. The supernatant is a soluble fraction in which the GST-fused recombinant peptide is present. This soluble fraction was mixed with Glutathione Sepharose 4B beads (GE Healthcare) and the GST-fused recombinant peptide was allowed to bind to the beads. The binding was carried out according to the protocol attached to the beads. The beads to which the GST-fused recombinant peptide was bound were washed several times with PBS and then mixed with PreScission Protease Cleavage Buffer (50 mM Tris-HCl, 15 0mM NaCl, 1 mM EDTA, and ImM DTT, pH 7.5) in which PreScission protease had been dissolved. The mixture was incubated at 5°C for 4 hours to cleave the recombinant peptides from GST. It should be noted that PreScission protease (GE Healthcare) is a GST fusion protein and is bound to the beads at the same time of the cleavage reaction. Next, the mixture was centrifuged at 5,000 g and 4°C for 5 minutes to recover the supernatant containing the recombinant peptides. The recovered recombinant peptide is hereinafter referred to as 39pr. Expression of the GST-fused recombinant peptide in the transformants was confirmed using SDS-PAGE shown in Fig. 2. A signal for the GST-fused recombinant peptide is indicated by a black arrow.

### <Example 2> Test for adherence of adhesive molecules of the present invention to glass

Two different peptide solutions were prepared: a 39pr solution of purified 39pr dissolved in the PreScission Protease Cleavage Buffer (50 mM Tris-HCl, 150 mM NaCl, 1 mM EDTA, and 1 mM DTT, pH7.5) and a peptide solution of BSA dissolved in purified water. The concentration of peptides was 0.2 mg/ml for both. 5 µl of the peptide solutions were each added dropwise onto a glass slide. Next, 1 µl of tyrosinase solution (1 mg/ml) prepared by dissolving lyophilized tyrosinase in potassium phosphate buffer (50 mM, pH6.5) was added to the dripped peptide solutions and gently mixed by pipetting. The glass slides were placed statically in an atmosphere of saturated water vapor at 22 to 25°C and incubated for 0 or 30 minutes. After the incubation, they were dried under vacuum at room temperature. Subsequently, the glass slides were washed for 30 minutes with shaking at 45 times/min. in distilled water. Then, they were dried under vacuum at room temperature again. After the drying, the glass slides were immersed in a 0.25% CBB (R-250) solution overnight and were then washed several times with distilled water. It was visually examined whether blue staining with the CBB staining was observed on the slides. If the peptide is bound to the surface of the glass, blue staining with the CBB staining should be observed.

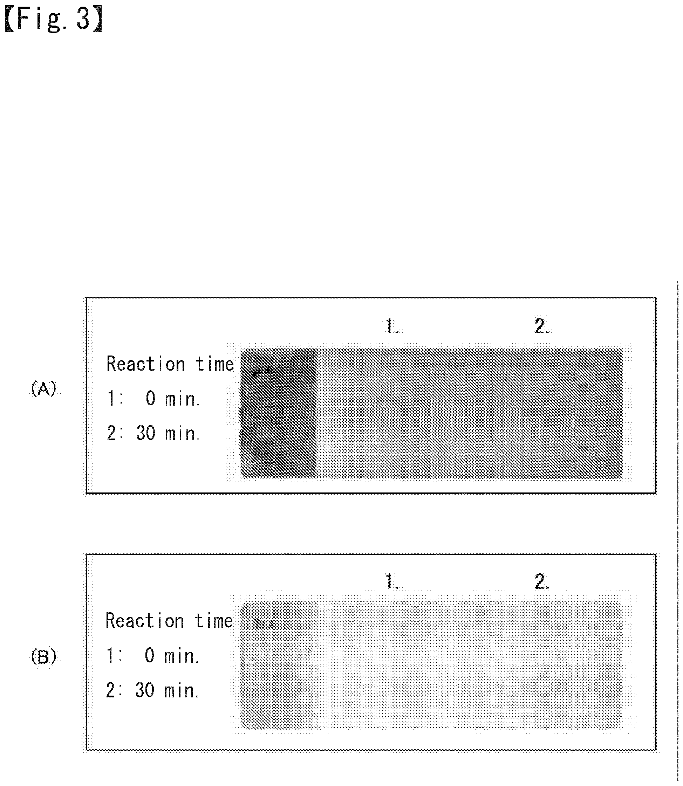

As shown in Fig. 3, BSA was not attached to the glass slide after the 30-minute washing (Fig. 3(B)). On the contrary, blue staining indicating that the 39pr was attached to the glass slide was observed even after the 30-minute washing, and stronger blue staining was observed after 39pr and tyrosinase were incubated for 30 minutes to convert tyrosine residues to DOPA, indicating that more peptides were bound to the glass slide (Fig. 3(A)).

## Claims

1. An adhesive molecule having an amino acid sequence of SEQ ID NO. 1, or an amino acid sequence of SEQ ID NO. 1 having a conservative amino acid substitution, a deletion, an insertion and/or a modification.

2. The adhesive molecule according to Claim 1, wherein the adhesive molecule contains at least one dihydroxyphenylalanine.

3. An adhesive comprising the adhesive molecule according to Claim 1 or 2.

4. A polynucleotide having a nucleotide sequence encoding the adhesive molecule according to Claim 1 or 2.

5. A vector comprising the polynucleotide according to Claim 4.

6. A method of bonding a first object to a second object, comprising applying the adhesive according to Claim 3 to a surface of the first object.

7. A method of coating a surface of an object comprising applying the adhesive according to Claim 3 to the surface of the object.

8. A method of enhancing adherence of an adhesive molecule comprising reacting tyrosinase with the adhesive molecule according to Claim 1.
